# EUROPEAN PATENT APPLICATION

(11) **EP 3 756 546 A1**
(43) Date of publication of application: **30.12.2020**
(21) Application number: 19182889.6
(22) Date of filing: 27.06.2019
(51) Int. Cl.: A61B 6/00

(54) **BONE TRABECULAE INDEX FOR X-RAY DARK-FIELD RADIOGRAPHY**

(71) Applicant: Koninklijke Philips N.V., 5656 AG Eindhoven (NL)
(72) Inventor: YAROSHENKO, Andriy, 5656 AE Eindhoven (NL); KOEHLER, Thomas, 5656 AE Eindhoven (NL)
(74) Representative: de Haan, Poul Erik

(57) **Abstract**

A method (200) and system (20) for expressing signals in a dark field X-ray image of bone (34; 44) in units of a trabecular quantity are disclosed, in which an X-ray dark field image of a bone having a trabecular network is acquired (204) at an image resolution that is not capable of resolving the trabecular network (41) of the bone. Information about the positioning of the scan bone relative to the X-ray dark field imaging apparatus used for acquisition is determined. Signals in the X-ray dark field image of the bone are converted (206) into a corresponding trabecular quantity, wherein the conversion accounts for the determined information about the positioning of the bone and depends on a plurality of generated X-ray dark field image signal normalization values, generated for a sample bone.

## Description

### FIELD OF THE INVENTION

The present invention relates to X-ray imaging in general and more particularly relates to dark-field X-ray imaging methods for quantifying bone trabeculae and X-ray imaging systems using the same.

### BACKGROUND OF THE INVENTION

Diagnosis of bone disorders such as osteoporosis is generally based on conventional X-ray imaging methods. Several qualitative risk indicators have been developed for the hand but quantitative measures therefor are still largely missing in clinical routine practice.

Peripheral quantitative CT (pQCT) is an emerging high-resolution X-ray imaging approach which aspires better diagnosis of bone disorders due to the insight gained into trabecular structures of the bone, which are known to be affected by many bone diseases. However, pQCT currently is only available to peripheral limbs which are easily accessible for CT scanning. The relatively high exposure to X-rays involved in high-resolution pQCT is another drawback of this method.

Another approach aiming at obtaining more information related to the trabecular structure of bone relies on the recent developments in the field of X-ray dark field imaging techniques and systems. Potdevin et al. "X-ray vector radiography for bone micro-architecture diagnostics", Phys. Med. Biol. 57, p. 3451-3461, 2012, describe an X-ray dark field imaging technique termed X-ray vector radiography (XVR) and apply it to obtain structural information on the trabecular network in hand bones and joints. They showed that an average mean orientation of bone trabeculae can be reliably obtained even from low resolution X-ray dark field radiographs that do not resolve the small features of the trabecular network. Jud et al. "Trabecular bone anisotropy imaging with a compact laser-undulator synchrotron x-ray source", Scientific Reports, vol. 7, article no. 14477, November 2017, further developed the XVR technique to generate bone trabeculae anisotropy measurements. These directional vector techniques, however, require the acquisition of multiple radiographs at many different bone orientations to produce accurate results for average mean orientation of bone trabeculae. Other quantitative risk indicators related to small features of the trabecular structure in bone which, in combination with the average mean orientation, would refine a diagnosis of bone related diseases are not described, but are desirable from the point of view of a practitioner in the medical field.

### SUMMARY OF THE INVENTION

It is an object of embodiments of the present invention to provide insight into the quantity of bone trabeculae from X-ray dark field images with a resolution, which, considered in isolation, are not resolving the small features of the trabecular network.

The above objective is accomplished by a method and device according to the present invention.

In accordance with one aspect of the invention, a method for expressing signals in a dark field X-ray image of bone in units of a trabecular quantity comprises acquiring an X-ray dark field image of a scan bone having a trabecular network. The acquisition is making use of an X-ray dark field imaging apparatus which provides the acquired X-ray dark field images of the scan bone at an image resolution that is not capable of resolving the trabecular network of the scan bone. Information regarding positioning of the scan bone is determined relative to a predetermined orientation of the X-ray dark field imaging apparatus used for acquisition. Signals in the X-ray dark field image of the scan bone are converted into a corresponding trabecular quantity, wherein the conversion depends on the determined information about the positioning of the scan bone and on a plurality of generated X-ray dark field image signal normalization values for a sample bone. Determining information regarding the positioning maybe determining information regarding the positioning of the bone in the x-ray beam with respect to e.g. an optical axis and a grating interferometer of the acquisition apparatus. Determining information regarding the positioning also may comprise determining information about an orientation of the scan bone relative to a predetermined orientation of the X-ray dark field imaging apparatus used for acquisition.

Multiple X-ray dark field images of the scan bone may be acquired at the same orientation of the scan bone and/or at different orientations. The step of converting signals in at least one X-ray dark field image of the scan bone into a corresponding trabecular quantity may comprise interpolating between at least two generated X-ray dark field image signal normalization values for the sample bone. Moreover, the method optionally comprises the further steps of determining a position of the scan bone relative to an optical axis of the X-ray dark field imaging apparatus and of rescaling signals in the acquired X-ray dark field image(s) of the scan bone, which rescaling is dependent on the determined position and is performed prior to converting the rescaled X-ray dark field image signals into a corresponding trabecular quantity.

The plurality of generated X-ray dark field image signal normalization values for a sample bone may be obtained through a calibration procedure, during which the at least the following steps are performed. In one step, an image of the sample bone at a resolution such that the trabecular network can be resolved is provided which thus resolves a trabecular network of the sample bone. In another step, a plurality of X-ray dark field images of the sample bone is provided, each X-ray dark field image of the sample bone corresponding to one of a plurality of different sample bone orientations, wherein the plurality of X-ray dark field images of the sample bone are provided at an image resolution such that the trabecular network is not resolved therein. Next, image processing means are used to perform image registration between the provided image at a resolution such that the trabecular network is resolved and each of the plurality of provided X-ray dark field images of the sample bone, thereby generating a correspondence between selected image areas of the image at a resolution at which the trabecular network is resolved and each one of the X-ray dark field images of the sample bone. Eventually, for each of the plurality of different sample bone orientation, an X-ray dark field image signal representative of a selected image area is normalized with a trabecular quantity to generate the plurality of X-ray dark field image signal normalization values. This trabecular quantity is obtained by the image processing means from the corresponding image area in the image at a resolution at which the trabecular network is resolved.

The image of the sample bone at a resolution at which the trabecular network is resolved, may be provided by acquiring an X-ray image at a resolution at which the trabecular network is resolved with a micro-CT or a peripheral CT scanner, for instance. Alternatively, or in combination thereto, the image of the sample bone at a resolution at which the trabecular network is resolved may be provided by way of a computer simulation of a sample bone comprising a trabecular network and a plurality of numerical X-ray scattering simulations for the computer-simulated sample bone are performed for a corresponding plurality of different computer-simulated sample bone orientations relative to a modelled grating interferometer of an X-ray dark field imaging apparatus. For such a computer simulation, the plurality of X-ray dark field images of the computer-simulated sample bone are numerically recorded at an image resolution such that the trabecular network is not resolved.

For calibration, each of the plurality of X-ray dark field images of the sample bone corresponding to a single sample bone orientation may be provided for a different position of the sample bone with respect to an optical axis of an X-ray dark field imaging apparatus. Hence, X-ray dark field images of the sample bone may be acquired at multiple sample bone orientations and multiple sample bone positions along the optical axis such that sample bone orientations are repeated at each sample bone position.

In another aspect, the present invention relates to a computer program comprising instructions which, when the program is executed by a computer, cause the computer to carry out at least the signal conversion of the method above, and preferably is also carrying out the signal rescaling.

In accordance with yet another aspect, a system for system for expressing signals in a dark field X-ray image of bone in units of a trabecular quantity includes an acquisition apparatus for acquiring an X-ray dark field image of bone material having a trabecular network. The X-ray dark field image of the bone material is acquired at an image resolution such that the trabecular network is not resolved. The system also comprises a tracking unit for tracking a position of the bone in the X-ray beam with respect to the acquisition apparatus, e.g. for tracking an orientation of the bone material relative to a predetermined orientation of the acquisition apparatus. At least one processing unit of the system is operatively connected to the tracking unit and the acquisition apparatus to respectively receive as inputs therefrom a tracking signal for the bone material and the X-ray dark field image of the bone material. Additionally, the at least one processing unit is configured for extracting information regarding the positioning of the bone material from the received tracking signal, for receiving a plurality of generated X-ray dark field image signal normalization values for a sample bone at different sample bone orientations with respect to the acquisition apparatus, and for converting signals in the received, acquired X-ray dark field image of the bone material into a corresponding trabecular quantity. This conversion of signals by the at least one processing unit uses the extracted orientation of the bone material and the received a plurality of generated X-ray dark field image signal normalization values as input variables for conversion.

The acquisition apparatus preferably comprises an X-ray imaging apparatus which includes an X-ray source, a grating interferometer and an X-ray detector, and the tracking unit is tracking an orientation of the bone material when imaged by the X-ray imaging apparatus. The tracked orientation is relative to an orientation of the grating interferometer. Additionally, the tracking unit may also be tracking a position of the bone material with respect to an optical axis of the acquisition apparatus. The tracking unit may comprise one or more of a tracking camera for tracking in three dimensions, a tape measure, image processing means for extracting orientational and/or positional information from a reference structure in an acquired X-ray image, and a bone support structure that generates a predetermined X-ray dark field signal when imaged by the acquisition apparatus. The tracking unit may actively determine an orientation and/or position of the bone material and transmit it to the at least one processing unit to be used directly, or the tracking unit may, in an alternative or additional manner, track an orientation and/or position of the bone material indirectly by performing indirect measurements, e.g. by recording images of the bone material and of a reference, and transmitting the measurement information to the at least one processing unit. The latter may then extract or determine the orientation and/or position of the bone material by well-defined pre-processing steps, e.g. image pre-processing. The at least one processing unit may further be adapted for rescaling signals in the acquired X-ray dark field image prior to converting the signals into a corresponding trabecular quantity. The degree of rescaling is determined by the position of the bone material with respect to an optical axis of the acquisition apparatus as tracked by the tracking unit.

It is an advantage of embodiments of the invention that X-ray dark field images and images displaying the amount of trabeculae can be obtained in conjunction with ordinary absorption X-ray radiographs and also with differential phase contrast radiographs. Improved contrast can be achieved through the absence of soft tissue signal contributions.

It is an advantage of embodiments of the invention that conventional X-ray tubes can be used. It is an advantage of embodiments of the present invention that the calibration technique also may be applied by normalizing for differences in voltages that are used. It is to be noted that the dependency between voltage and dark-field signal is not linear, since doubling the voltage does not double the mean energy. In some embodiments, the normalization therefore may be performed for a number of voltages and the voltage used thus may be taken into account when applying the normalization.

It is an advantage of embodiments of the invention that a large field of view can be imaged, assessed in terms of trabecular quantity and displayed, e.g. a large portion or the whole of a subject hand can be visualized.

It is an advantage of embodiments of the invention that a large variety of a subject's scanned bone postures are accommodated, which benefits elderly people with restricted mobility.

It is an advantage of embodiments of the invention that orientation and/or position tracking of a scan bone allows for fewer exposures to X-rays, reducing the overall absorbed dose.

It is an advantage of embodiments of the invention that orientation and/or position tracking of a sample bone allows for an accurate calibration of the acquired X-ray dark field image signals in terms of trabecular quantity.

It is an advantage of embodiments of the invention that a quantitative risk indicator for assisting in the diagnosis of bone disorders by a healthcare professional is readily provided. The quantitive risk indicator can be combined with other morphological risk indicators, which can be of quantitive or qualitative nature.

It is an advantage of embodiments of the invention that the amount of trabeculae in bone can be assessed in body regions which are not peripheral and more difficult to scan by means of compact pQCT scanners.

It is an advantage of embodiments of the invention that the amount of trabeculae in bone can be measured at regular intervals, thereby enabling the study of time-varying changes in the amount of trabeculae.

It is an advantage of embodiments of the invention that a good reference trabecular bone structure can be provided and studied numerically by simulation. This allows for less demanding equipment as compared to a physical reference bone and X-ray dark field imaging system. It also allows for a very flexible way of adding or removing experimental restrictions into the simulation model.

Particular and preferred aspects of the invention are set out in the accompanying independent and dependent claims. Features from the dependent claims may be combined with features of the independent claims and with features of other dependent claims as appropriate and not merely as explicitly set out in the claims.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

### BRIEF DESCRIPTION OF THE DRAWINGS

The invention will now be described further, by way of example, with reference to the accompanying drawings, in which:
Fig. 1 is a flowchart relating to a calibration method for generating a plurality of X-ray dark field image signal normalization values, in accordance with an embodiment of the present invention.
Fig. 2 is a flowchart illustrating method steps for expressing signals in a dark field X-ray image of bone in units of a trabecular quantity, in accordance with an embodiment of the present invention.
Fig. 3 illustrates schematically an embodiment of a system that is adapted for carrying out the method steps for expressing signals in a dark field X-ray image of bone in units of a trabecular quantity.
Fig. 4 illustrates schematically a bone comprising a trabecular network.

The drawings are only schematic and are non-limiting. In the drawings, the size of some of the elements may be exaggerated and not drawn on scale for illustrative purposes. The dimensions and the relative dimensions do not necessarily correspond to actual reductions to practice of the invention.

Any reference signs in the claims shall not be construed as limiting the scope.

### DETAILED DESCRIPTION OF ILLUSTRATIVE EMBODIMENTS

The present invention will be described with respect to particular embodiments and with reference to certain drawings but the invention is not limited thereto but only by the claims.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

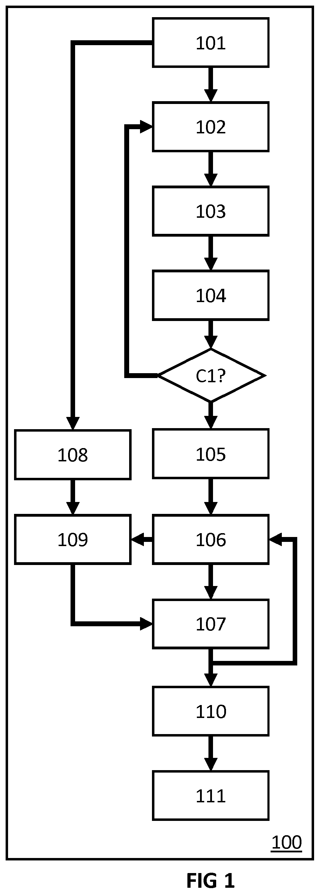

With reference to Fig. 1, an exemplary calibration method 100 for generating a plurality of X-ray dark field image signal normalization values for a sample bone is first described. These signal normalization values serve as inputs to the signal conversion step during a subsequent bone scan for which the conversion of signals in an acquired X-ray dark field image into a trabecular quantity is sought after. The calibration method 100 may start by providing a sample bone in a first step 101. This sample bone can be a physical human or animal bone (e.g. cadaver hand, femur) or a synthetic bone mimic natural bone shapes and materials, for example, and comprises a trabecular network.

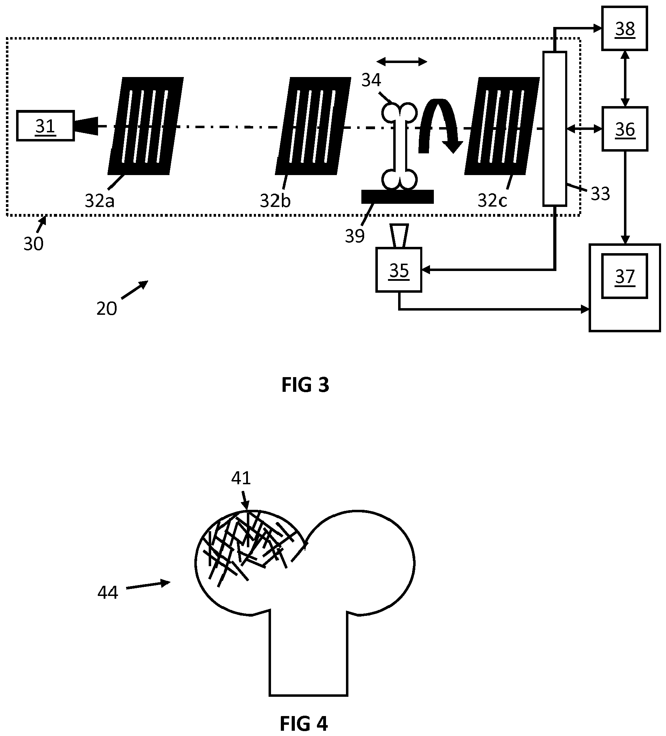

Referring briefly to Fig. 4, part of a natural or artificial bone 44 is schematically illustrated. Typically, a bone 44 has a harder, more dense outer layer, also referred to as cortical bone, which provides the bone's 44 supportive and protective functions. An inner, less dense tissue, also referred to as cancellous bone, includes a porous network at length scales of the order of tens to hundreds of micrometers (e.g. trabecular thickness from about 40 µm to about 200 µm and trabecular spacing from about 300 µm to about 800 µm) - the trabecular network 41. The geometry and density of the trabecular network directly influences the bone's elastic modulus and stiffness and thus is of uttermost importance for the bone's 44 capability to sustain loads and withstand stress-induced fracture. Therefore, an erosion of the trabecular network structure 41 in cancellous bone, associated with a loss of trabecular bone mass, e.g. by thinning of the struts and/or plates making up the trabecular network 41, their disappearance or cracks therein, is a clinically relevant process since it may cause osteopenia or even osteoporosis. The latter two bone disorders greatly increase the subject's bone fracture risk. Hence, the correct quantification of the bone trabeculae in units of trabecular quantity is a clinically relevant factor for fracture risk assessment and/or the diagnosis of bone diseases, disorders or anomalies such as osteopenia, osteoporosis, osteoarthritis, osteophytes, etc. Other quantitative or qualitative factors may be taken into account as well to comfort a diagnosis by a medical practitioner. In the clinical field of rheumatology, for instance, there has been a continuous, long-lasting effort to move toward a commonly acknowledged reference method for scoring conventional radiographs of subchondral bone and joint spaces in hands and feet (subchondral trabecular bone is predominant near joints and is of relevance in collecting evidence for osteoarthritis). One of which is the Sharp/van der Heijde method proposed by D. van der Heijde "How to read radiographs according to the Sharp/van der Heijde method", Journal of Rheumatology 2000; 27:261-3 or the simplified alternative thereof, the Simple Erosion Narrowing Score (SENS) method, described in van der Heijde et al. "Reliability and sensitivity to change of a simplification of the Sharp/van der Heijde radiological assessment in rheumatoid arthritis", Rheumatology (Oxford) 1999; 38:941-7. These methods require an appropriate training to minimize reader disagreement and is susceptible to inter-/intra-observer variations. They also assign discrete scores to a continuum of joint damages. This shows that is still a need for harmonized and less subjective assessment methods. Expressing radiographic images of the hands or feet in units of a trabecular quantity as an objectively measured quantitative indicator is recognizes this need and offers a solution. Currently available quantitative imaging techniques such as in-vivo areal or volumetric dual energy X-ray absorptiometry (DEXA), when used to obtain a bone mineral density (BMD) value, are often affected by large uncertainties, which makes a reliable diagnosis based on quantitive DEXA measurements challenging. This difficulty is linked to the correct bone width estimation and is further complicated various intra-/extraosseous X-ray absorption effects on the other hand. For instance, the spaces of bone trabeculae are generally filled with bone marrow in living beings, the exact composition of which is often unknown. Magnetic resonance imaging (MRI) is giving more insight into the bone marrow composition and volume, but is often unavailable or expensive to obtain. The lacking contrast between the bone marrow and the trabecular bone and the inherently small length scales of the trabecular network are obstacles that are a hindrance to the adoption of measuring the amount of trabeculae. For instance, the trabecular network structure is generally not resolvable in conventional computed tomography (CT) scanners which bars them from gaining direct insight into the trabecular quantity. Micro-CT scans or synchrotron X-ray sources of high brilliance may be used for resolving these small length scales, but are associated with an exposure to high doses of ionizing radiation and a reduced field of view. Peripheral quantitative CT (pQCT) is offering an improved field of view, but still requires multiple exposures corresponding to different projection views and is restricted to the scan of limbs. It is thus an advantage of embodiments of the present invention, which provide X-ray dark field images of bone, to gain insight into the trabecular quantity without relying on scanning methods operating at a resolution at which the trabecular network is resolved. In consequence, this brings the trabecular quantity as clinical risk factor into the reach of clinical imaging techniques using low-brilliance, polychromatic sources. Large field of views are available, which benefits patients because a larger region of interest may be imaged without requiring the repeated imaging of smaller fields which, in combination, provide the larger field.

Referring again to Fig. 1, an image of the sample bone is provided in another step 108. The image resolution of the provided image is such that the trabecular network 41 of the sample bone is resolved. One way to obtain the image of the sample bone at a resolution at which the trabecular network is resolved is to perform a micro-CT scan (e.g. fan beam or cone beam) or a peripheral CT scan of the sample bone. Available micro-CT scanners resolve spatial features below 100 micron and may even resolve submicron features. As the calibration is performed for a sample bone, an exposure to a higher dose is not a safety risk for the subject (e.g. patient) during a later subject bone scan using the plurality of X-ray dark field image signal normalization values obtained at the end of the calibration. The images of the sample bone at a resolution at which the trabecular network is resolved, which serve as a calibration standard, may also be obtained or complemented by X-ray imaging with a highly collimated, monoenergetic synchrotron X-ray source. In yet another step, a plurality of X-ray dark field images of the sample bone are provided 104, e.g. by acquiring a plurality of X-ray projection images by means of an X-ray dark field imaging apparatus. The plurality of X-ray dark field images of the sample bone are provided at an image resolution that does not spatially resolve the trabecular network 41 of the sample bone. This may happen before, after or even simultaneously to the scan. An example of an embodiment for which the scan and the acquisition of the plurality of X-ray dark field images is performed simultaneously may be a multi-modal X-ray imaging apparatus with different resolution settings and/or the possibility to average or down-sample images with a given resolution to lower image resolution. In some embodiments, each of the plurality of provided X-ray dark field images 104 is corresponding to a particular sample bone orientation and/or a particular sample bone position. The sample bone orientation may be set or updated 103, independently of the setting or updating of the sample bone position 102. For instance, an X-ray dark field image is acquired repeatedly as long as a condition C1 is not met. Before each new X-ray dark field image acquisition, a sample bone orientation 103 and/or sample bone position 102 may be adjusted. It is also possible to repeatedly acquire X-ray dark field image without adjusting the sample bone orientation and/or position, e.g. for the purpose of averaging multiple acquisitions to reduce noise. The acquisition of the plurality of X-ray dark field images stops if the condition C1 is fulfilled, for instance, if all the sample bone orientations in a predetermined list of different sample bone orientations have been set 103, if all the sample bone positions in a predetermined list of different sample bone positions have been set 102, or both.

The acquisition of X-ray dark field images of bone in general, including the acquisition of X-ray dark field images of the sample bone and of scan bone (e.g. a patient's bone, e.g. hand or feet), is now described in more detail with reference to Fig. 3, in which an embodiment of a system 20 for expressing signals in a dark field X-ray image of bone in units of a trabecular quantity is shown schematically. The system 20 comprises an acquisition apparatus 30, which maybe an X-ray imaging apparatus including an X-ray source 31, an X-ray detector 33 and a grating interferometer 32a-c. The presence of the grating interferometer 32a-c allows for the acquisition of X-ray dark field images, e.g. images obtained by X-ray projections for which only the scattered X-ray photons are considered. Similar to phase-contrast X-ray imaging, dark field X-ray imaging is phase sensitive, i.e. sensitive to changes in the real part of the refractive index for X-ray radiation, e.g. changes in the electron density, rather than to the imaginary part, which is linked to absorption. This has the advantage that a visible contrast for interfaces and edges, causing more pronounced reflection and diffraction of X-rays, is enhanced in X-ray dark field images as compared to conventional X-ray absorption radiography directed to the study of absorption in the forward beam. Hence, weakly absorbing soft-tissue such as skin, muscles, ligaments, tendons, etc., surrounding the bone give rise to stronger signals. This facilitates the definition of a soft-tissue-bone boundary for instance, which is of advantage also in a (boundary) edge-based image registration step. Furthermore, microscopic inhomogeneities such as the porous network of bone trabeculae are generating (ultra-) small angular scattered X-ray signals that are probed by dark field imaging. Therefore, X-ray dark field imaging as compared to conventional absorption imaging, reveals structural information beyond the resolution limits of the detector, e.g. sub-pixel structural information.

The X-ray source 31 may be a compact, low-brilliance, polychromatic source, e.g. an X-ray source used in conventional CT, and the detector 33 may be a Si photodiode array, a CCD or CMOS X-ray image sensor, or a flat panel detector comprising a pixel array. In this particular embodiment, the grating interferometer 32a-c comprises three gratings 32a, 32b and 32c, each comprising a plurality of parallelly running grating lines. The first grating or source grating 32a is placed in front of the X-ray source 31, between the source 31 and the detector 33, and mimics multiple coherent X-ray slit sources for X-ray radiation emitted by the source 31 and transmitted through the first grating 32a. It follows that the first grating 32a is optional if the X-ray source 31 is already satisfying the requirements on spatial coherence or if spatial coherence is ensured by other means. The first grating 32a may be an absorption grating comprising a plurality of transmissive grating lines. The coherence of the transmitted X-ray radiation is exploited by the second grating 32b, positioned between the first grating 32a and the detector 33 to generate a Talbot carpet. The second grating 32b may be a weakly absorbing phase grating comprising a plurality of grating lines causing strong phase shifts for coherent X-ray radiation passing through it. The periodic intensity pattern at a predetermined Talbot order (or fractional order) is analysed by the third (analyser) grating 32c, which is positioned at an axial distance from the second grating 32b at which that Talbot order occurs. Here, the distance is measured with respect to an optical axis of the system 20 (dash-dotted line in Fig. 3). The third grating 32c typically is an absorption grating comprising a plurality of transmissive grating lines, periodically arranged with a spatial line period that matches the spatial period of the predetermined Talbot order. In the absence of any disturbance in the propagation path of the X-ray radiation toward the detector 33, the detector 33 thus detects a strong signal, preferably the maximum signal. If a scattering object such as bone 34 is present in the X-ray path, e.g. between the second and the third grating 32b, 32c or in front of the second grating 32b between the first and the second grating 32a, 32b, this causes a disturbance in the periodic behaviour of the predetermined Talbot order, e.g. causing a lateral shift thereof, such that less X-ray radiation is reaching the detector 33 through the analysing third grating 32c, which now partially blocks the disturbed (e.g. shifted) X-ray intensity pattern. A weaker signal is thus detected by the detector 33 in the presence of a scattering object. Phase stepping techniques may be applied, e.g. by stepping a transversal position of the third grating 32c (e.g. in a transversal direction perpendicular to the optical axis and to the grating lines). This results in a periodic detector signal for each detector pixel element, regardless of the scattering object (e.g. bone 34) is present or absent. The periodic, phased-stepped weaker detector signals in the presence of the scattering object and the periodic, phased-stepped stronger reference signal in the absence of any scattering object may then be expanded into a Fourier series, e.g. by performing a discrete Fourier transform to obtain a series of Fourier coefficients a0, a1, ..., and b0, b1,..., for the presence and the absence of the scattering object, respectively. The ratio of the mean-normalized first Fourier coefficients, e.g. V[m,n] = (a1[m,n]/a0[m,n])/(b1[m,n]/b0[m,n]), provides a visibility or contrast measure for each detector pixel element of the m-th row and n-th column of the detector 33, which may be used to represent the X-ray dark field image. It is noted that in this particular embodiment, the phase stepping implies that a plurality of X-ray projection images are acquired by the detector 33 to acquire one X-ray dark field image. However, it is also possible to obtain the X-ray dark field image from a single projection image acquired by the detector 33 if the visibility is determined for a well-aligned, non-stepped third grating 32c on the basis of the weaker signal detected by the detector 33 in the presence of the scattering object and the previously recorded and stored, stronger reference signal detected by the detector 33 in the absence of any scattering object.

The grating lines in each of the three gratings 32a-c typically have a preferred direction, e.g. the direction in which the lines extend, although grid-like apertures with lines oriented along two orthogonal directions may also be used in practise. In consequence of a preferred orientation of the grating lines, the grating interferometer 32a-c as a whole is most sensitive to scattering perpendicular to the preferred orientation of the grating lines, but is blurring scattering information along the direction of the grating lines. Thus, unless 2D-gratings are implemented or the scattering object in an isotropic scatter object, it is recommendable to acquire X-ray dark field images with respect to a plurality of different sample bone orientations 103 in order to retrieve a more complete X-ray dark field image data set. In particular, highly anisotropic scattering objects or scattering objects with a varying degree of anisotropy, as it is known to be the case for trabecular bone, are characterized in a more complete way during calibration purposes if a plurality of object (e.g. sample bone) orientations are selected for corresponding X-ray dark field image acquisitions. Here, different sample bone orientations may be defined with respect to the preferred direction of the grating interferometer 32a-c, for instance, the sample bone 34 may be rotated relative to the grating interferometer 32a-c. This may be achieved by either rotating the three gratings 32a-c about the optical axis, leaving the sample bone 34 fixed or by rotating the sample bone 34 about the optical axis, leaving the gratings 32a-c fixed. The latter is illustrated in Fig. 3, in which the sample bone 34 is mounted on a bone support structure 39, e.g. a rotation stage for rotating the bone around the optical axis. In view of the magnifying effect of the acquisition apparatus 30 described above, it is also preferable to acquire X-ray dark field images of the sample bone for each of a plurality of sample bone positions 102 along the optical axis during calibration, e.g. by moving the sample bone 34 forth or back in the direction of the optical axis, e.g. by moving the bone support structure 39 forth or back in the direction of the optical axis. Grating line widths and grating line periods for each of the three gratings 32a-c, as well as the respective axial distances between them, depend on the required image resolution, the pixel pitch of the X-ray detector 33, the level of magnification, etc., and are determined and/or optimized by the skilled person according to known methods and/or through simulation. The X-ray imaging apparatus with a grating interferometer 32a-c is only one example of an acquisition apparatus that is adapted for acquiring X-ray dark field images of bone. The skilled person is aware of the different approaches to X-ray dark field imaging or X-ray phase-contrast imaging from which X-ray dark field signals are obtainable and will adapt the system and methods described herein accordingly. A review of various X-ray imaging techniques providing phase-contrast and dark field signals is compiled in Zhou et al. "Development of phase-contrast X-ray imaging techniques and potential medical application", Physica Medica, vol. 24, issue 3 (2008), pp. 129-148; and for contributions to Talbot interferometry and the use of low-brilliance sources reference is made to Pfeiffer et al. "Phase retrieval and differential phase-contrast imaging with low-brilliance X-ray sources", Nature Physics, vol. 2 (2006), pp. 258-261, Pfeiffer et al. "Hard X-ray dark-field imaging using a grating interferometer", Nature Materials, vol. 7 (2008), pp. 134-137, Momose et al. "Phase Tomography by X-ray Talbot Interferometry for Biological Imaging", Japanese Journal of Applied Physics, vol. 45 (2006), pp. 5254-5262, and Momose et al. "Sensitivity of X-ray Phase Imaging Based on Talbot Interferometry", Japanese Journal of Applied Physics, vol. 47 (2008), pp. 8077-8080. If taken into consideration, these techniques, which are not repeated here, will instruct the skilled artisan to construe quantity of alternative embodiments. For example, whereas embodiments of the present invention are illustrated for X-ray dark field images, embodiments wherein the X-ray dark field images are derived from the differential phase-contrast images also could be used, since the x-ray dark field signal is proportional to the noise (standard deviation) in differential phase-contrast image.

Referring back to the embodiment of Fig. 1, image processing means are used to perform image registration 105 between the provided image 108 with a resolution such that the trabecular network can be resolved and each of the plurality of provided X-ray dark field images 104 of the sample bone. The image registration step 105 thus generates a correspondence between selected image areas for the image of the sample bone at a resolution such that the trabecular network can be resolved and each one of the provided X-ray dark field images of the sample bone with resolution at which the trabecular network cannot be resolved, wherein selected areas may correspond to the whole image or sub-areas therein, e.g. to one or more bones or joints of a limb. The image registration step 105 may correlate the intensity information the image of the sample bone at a resolution at which the trabecular network is resolved and each one of the provided X-ray dark field images of the sample bone at a resolution at which the trabecular network is not resolved, or geometric features such as lines or shapes, or a combination of both. Image processing means may be applied to the images to detect and correlate the geometric features, e.g. lines or shapes, which image processing means may encompass the application of suitable edge filters, averaging filters, morphological image processing routines such as erosion, dilation, opening and closing, etc. Available image registration methods may be use too, e.g. Woods' automated image registration or mutual information. Optimal alignment of the registered images may under a given feature space, search space and search strategy is generally assessed by a measure of similarity, e.g. pixel intensity differences, deformation energy cost, etc., for which an optimal aligning transformation is produced. Alignment transformations are usually parametrized and may involve rigid, linear and affine geometrical transformations including scaling, rotation and translation, or non-rigid, elastic transformation such as warping/distortion, diffeomorphisms and flow. The image processing means used for image registration may be performed by one or more processing units 36 of the system 20 shown in Fig. 3. The one or more processing units 36 may also control the image acquisition of the detector 33, the sample bone orientations and positions via the bone support structure 39, the graphical output of images to a connected display unit 37, the storage and retrieval of acquired X-ray dark field images to a storage unit 38, etc. The one or more processing units 36 and the storage unit 38 maybe provided in a local processing device, e.g. a client computer at the premises where the system 20 is installed, or maybe provided in a distributed or remote fashion, e.g. as server-based or cloud-based services (e.g. remote processing units and storage units, accessed wire a network or communication link).

After a completed image registration 105, one or more regions of interest may be selected 106 for further image analysis, in particular for the assessment of trabecular quantity, e.g. measured by the number of trabecular interfaces or the number of trabecular (struts) per mm. This selection may be done in an automated and/or expert-guided way in the plurality of X-ray dark field images and is shared with the image processing means that is used to analyse the trabecular quantity in the corresponding selected region(s) of interest in the image 109 at resolution such that the trabecular network can be resolved. For instance, an automated and/or expert-guided selection of region(s) of interest may be directed to a particular hand bone or bone region, e.g. subchondral bone, or even to a single pixel, for which a strong X-ray dark field signal is obtained. With respect to the system 20 in Fig. 3, the selection may be performed by an expert via a graphical user interface on a display unit 37, e.g. touch screen or panel, remote desktop (screen), portable graphic displays such as smart phones or tablets, etc., whereas automated selections may be carried out by the one or more processing units 36. In contrast to micro-CT bone scans, for which random projections are used to obtain averaged means and ranges for typical trabecular indices such as trabecular thickness, trabecular spacing or bone volume density, the present calibration takes advantage of the fact that a corresponding determined orientation for each X-ray dark field image of the sample bone is available. Therefore, the image processing means more accurately determine a trabecular quantity 109 for the sample bone as a function of sample bone orientation in the corresponding selected region(s) of interest of the image at a resolution such that the trabecular network can be resolved. This duly accounts for the anisotropic nature of the trabecular network 41.

In some embodiments, the normalised scatter, i.e. the dark-field signal divided by the transmission, can be determined which gives an idea of how much is absorbed per scattering unit.

For example, the image processing means may determine a trabecular quantity 109 in a corresponding selected region of interest of the image at a resolution such that the trabecular network can be resolved along the determined sample bone orientation by counting the number of times trabecular bone structures, e.g. struts, are crossed along a plurality of parallel lines oriented according to the determined sample bone orientation and intersecting that region of interest. Although a trabecular quantity is preferably determined, also other related trabecular indicators may be quantified in a similar manner, e.g. mean trabecular thickness and/or trabecular spacing for a sample bone orientation. According to the embodiment of Fig. 1, the X-ray dark field image signal representative of a selected image area (e.g. an X-ray dark field image signal representing a single pixel intensity value of the dark field image or an X-ray dark field image signal representing an averaged pixel intensity value of the selected area of the dark field image) is normalized 107 with the trabecular quantity obtained by the image processing means from the corresponding image area in the image at a resolution such that the trabecular network can be resolved. This normalization is performed for each of the plurality of different sample bone orientations and may be repeated for each selected region of interest. The normalization assigns a trabecular quantity for each sample bone orientation to the X-ray dark field image signal representative of the selected image area, for instance, the normalization may assign a trabecular quantity to each unique X-ray dark field image signal within an X-ray dark field image for a first sample bone orientation and then assign a trabecular quantity to the X-ray dark field image signals at the same locations as each of the unique X-ray dark field image signals for each further sample bone orientation. The trabecular quantity assigned by the normalization may be the result of averaging over one or more selected regions of interest adjacent to or overlapping with the selected image area. The trabecular quantity assigned by the normalization may further be the result of averaging over one or more nearby intermediate sample bone orientations (e.g. fine-grained sample bone orientations around each sample bone orientation step in a coarser sample bone orientation scan. As a result of the normalization, a plurality of X-ray dark field image signal normalization values are generated 110, e.g. in the form of a look-up table for calibration or based on target-value-pairs on a linear or polynomial fitting curve, parametrized by the different sample bone orientations (and optionally sample bone positions). This plurality of generated X-ray dark field image signal normalization values is stored on a data carrier, e.g. USB stick, CD, DVD, etc., or on a storage unit, e.g. the storage unit 38 in Fig. 3, which may be a local memory unit of the system 20 or a remote server-based storage location. The stored plurality of generated X-ray dark field image signal normalization values may then be retrieved at a later stage from the data carrier (or a copy thereof), or may be communicated at a later time to the client device if stored at a remote location (e.g. over a communication/network link, e.g. the Internet or private network).

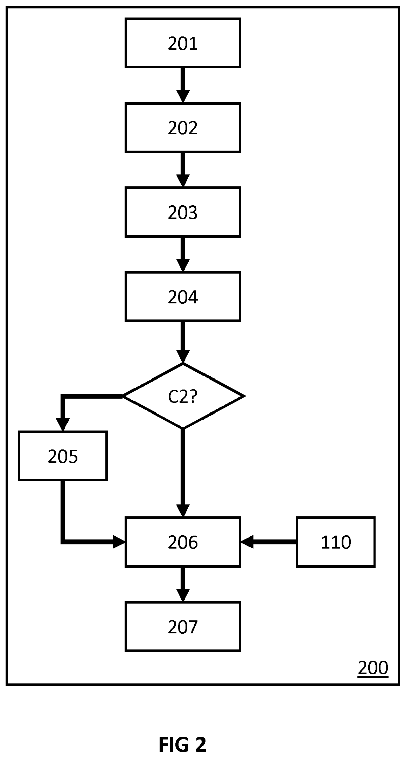

With reference to Fig. 2, an exemplary embodiment 200 for expressing signals in a dark field X-ray image of bone in units of a trabecular quantity is described. In this particular embodiment, the generated plurality of X-ray dark field image signal normalization values from the calibration procedure are used to convert X-ray dark field image signals into units of trabecular quantity. In a first step, a scan bone is provided 201, e.g. a patient's hand bone for which X-ray dark field images are subsequently acquired. This step may include placing and orienting the scan bone on a bone support structure 39, e.g. pushing a hand against the support structure and securing it with straps or tape after a first orientational repositioning with respect to a reference mark on the support structure, for instance. Next, information regarding a scan bone positioning, e.g. a scan bone orientation of the scan bone is determined 202 and preferably also a scan bone relative position 203. The information regarding the scan bone positioning, e.g. the scan bone orientation and scan bone position are determined with respect to a predetermined orientation of an acquisition apparatus for acquiring X-ray dark field images, e.g. with respect to the preferred orientation of the grating interferometer 32a-c and the optical axis of the acquisition apparatus 30 previously described with reference to Fig. 3. A tracking unit, e.g. the tracking unit 35 shown in Fig. 3, maybe provided to directly or indirectly allow determining the scan bone orientation and, preferably, also the scan bone position. For instance, a tape measure or a tracking camera may be used as a tracking unit. Clinical staff may read off the scan bone orientation or position from the tape measure and enter it into the system 20 (e.g. via a user interface); or the tracking camera may be used to track the patient's limb orientation/position or that of an adjacent reference mark on the bone support structure 39 in three dimensions (e.g. by shape recognition and 3D localization). The so determined scan bone orientation and preferably scan bone position are sent by the tracking unit to the one or more processing units 36 as input parameters. It is also possible to send indirectly obtained information on the scan bone orientation/position, e.g. as camera images acquired by the tracking unit, to the one or more processing units 36, which then extracts therefrom the required scan bone orientation/position. Alternatively, or additionally, the bone support structure 39 may have incorporated into it or attached to it, geometrically shaped (e.g. cross-shaped or triangularly shaped or quadrilateral shaped) reference structures, e.g. incorporated or attached to the bone support structure 39 in a region that is not obstructed by the scan bone or subject limb. The one or more processing units 36 may then be programmed to determine a scan bone orientation/position based on image analysis of the X-ray dark field image acquired by the detector 33, e.g. by analysing the scan bone shape and area in the X-ray dark field image or by analysing the projected reference structure in the X-ray dark field image and comparing it to a standard bone shape and area or to a standard projection of the reference structure. Deviations may then be quantified, which allow the determination of the scan bone orientation/position (e.g. using stereographic projection models). In a further step, an X-ray dark field image of the scan bone is acquired 204 by the acquisition apparatus 30. The acquisition step may be performed before, after or at the same time as the scan bone orientation/position step. The X-ray dark field image acquired by the acquisition apparatus 30 is characterised by an image resolution which does not resolve the trabecular network 41 of the scan bone. Next, the one or more processing units 36 or a clinical staff may check whether an imaging condition C2 is met. If the condition C2 is not met, the acquired X-ray dark field images is rescaled 205 before proceeding to the signal conversion step 206, otherwise such a rescaling step 205 is skipped. The condition C2 typically depends on the determined scan bone position 203; the condition is met if the determined scan bone position agrees within tolerances with a reference position of the sample bone, otherwise rescaling corrects for the magnification effects caused by a mismatch of the same and the scaling of the x-ray dark-field signal, as it grows linearly with the distance between the sample and the grating. Next, signals in the X-ray dark field image of the scan bone are converted into a corresponding units of trabecular quantity 206. This conversion is based on the determined positioning information, e.g. the orientation of the scan bone, and the plurality of generated X-ray dark field image signal normalization values 110. For instance, the on or more processing units 36 may send a request to the storage unit 38 of the system 20 to retrieve the generated X-ray dark field image signal normalization values for the determined scan bone orientation (and preferably scan bone position), e.g. from a stored look-up table. If the plurality of generated X-ray dark field image signal normalization values 110 is only stored for sample bone orientations/positions that differ from the currently determined scan bone orientation/position, the generated X-ray dark field image signal normalization values 110 for the two, three or more closest available sample bone orientations/positions may be loaded for 1D or 2D interpolation. Then, the interpolated X-ray dark field image signal normalization values are used for the signal conversion. The converted X-ray dark field image signal may correspond to intensity value of a pixel in the dark field image and the complete dark field image may be converted and displayed 207, e.g. on the display unit 37. However, also X-ray dark field image signals corresponding to an average over pixel intensity values in the dark field image may be converted into units of trabecular quantity and displayed 207, e.g. to improve image quality by reducing noise. The converted X-ray dark field image may be displayed 207 next to a conventional X-ray absorption radiograph of the scan bone or displayed as an overlay thereto.

Expressing the X-ray dark field image signals in units of trabecular quantity does not require dedicated training of health care professionals to derive a score as bone disease risk factor. It shows the distribution of trabecular quantity almost instantaneously and allows for an earlier diagnosis of bone diseases or disorders, for instance the erosion of bone trabeculae by displaying a reduced amount of trabeculae. Subject bone scans can be repeated in intervals to assess bone disease progression or to assess promising treatments. Embodiments of the present invention may also apply to other fields, for instance to lead quantitive studies in X-ray dark field imaged alveoli of the lung, to test the application of Wolff's law, to assess bone strength in joint modelling, to study load distribution changes with age, to correlate bone trabeculae with bone marrow measurements, to assessing degrees of differentiation in species-related studies with impact in anthropology or archeology, etc. While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. The invention is not limited to the disclosed embodiments.

For example, it is possible to provide an image of sample bone 108 at an image resolution that resolves the trabecular network 41 by undertaking a computer simulation. The trabecular network structure may be modeled as a three-dimensional structure comprising bone material voxels and void or bone marrow voxels. Typical size distributions and/or orientations for trabecular struts and pores may be based on existing studies, e.g. from pQCT or micro-CT studies (in-vivo/ex-vivo) of limbs. Then X-ray dark field images may be generated by simulating the propagation and detection of X-ray radiation through the modelled trabecular network at different orientations. Here, the different sample bone orientations may correspond to orientations relative to a simulated grating interferometer (e.g. according to the specifications of a physical acquisition apparatus 30). However, the different sample bone orientations may also correspond to orientations relative to a simulated optical axis along which the simulated coherent X-ray radiation is propagating since the X-ray dark field signal may be detected directly in a numerical computer simulation (e.g. by rejecting un-scattered, forward propagating X-rays transmitted through the trabecular bone model as simulation outputs, e.g. by setting an angular rejection threshold for scattered simulated X-rays). It is noteworthy to mention that the plurality of X-ray dark field images may thus also provided numerically if a recorded resolution in such a computer simulated X-ray scatter experiment is set low enough to not resolve the features of the trabecular network 41 simulated. This may also be achieved by down-sampling or averaging an X-ray dark field image obtained from simulation.

A computer program may be conceived and distributed, which comprises a set of instructions, which when executed by a computing device perform one or more of the method steps, preferably in conjunction with inputs from the acquisition apparatus 30, e.g. X-ray dark field image inputs. The computer program is thus contrived to perform the conversion step 206 for received X-ray dark field image input and generated X-ray normalization values 110, which are also received as inputs or provided within the program. The computer program preferably also comprises instruction for rescaling received X-ray dark field image input, taking a further (user) input for the scan bone position into account. Moreover, the computer program may comprise instruction for performing one or more step of a computer simulation as described in the foregoing paragraph.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method (200) for expressing signals in a dark field X-ray image of bone in units of a trabecular quantity, comprising:
- acquiring an X-ray dark field image of a scan bone having a trabecular network using an X-ray dark field imaging apparatus (204), the acquired X-ray dark field image of the scan bone being provided at an image resolution such that the trabecular network is not resolved, determining information about the positioning of the scan bone with respect to the X-ray dark field imaging apparatus used for acquisition, and
- converting (206) signals in the X-ray dark field image of the scan bone into a corresponding trabecular quantity, based on the determined information about the positioning of the scan bone and a plurality of generated X-ray dark field image signal normalization values (110) for a sample bone.

2. A method (200) according to claim 1, wherein said determining information about the positioning comprises determining information about an orientation (202) of the scan bone relative to a predetermined orientation of the X-ray dark field imaging apparatus used for acquisition.

3. A method according to any of the previous claims, the method further comprising:
- determining a position (203) of the scan bone relative to an optical axis of the X-ray dark field imaging apparatus, and
- rescaling (205) signals in the X-ray dark field image of the scan bone based on the determined position and prior to converting (206) the rescaled signals into a corresponding trabecular quantity.

4. A method according to any one of the previous claims, wherein the method further comprises:
- providing a resolution image of the sample bone (108) at an image resolution resolving the trabecular network of the sample bone,
- providing one or more X-ray dark field images of the sample bone (104) at a corresponding one or more sample bone orientations (103), the one or more X-ray dark field images of the sample bone being provided at an image resolution such that the trabecular network is not resolved,
- using image processing means to perform image registration (105) between the provided resolution image at an image resolution resolving the trabecular network and the one or more provided X-ray dark field images of the sample bone so as to generate a correspondence between selected image areas, and
- normalizing (107) an X-ray dark field image signal representative of a selected image area (106) with a trabecular quantity obtained by the image processing means (109) from the corresponding image area in the resolution image at an image resolution resolving the trabecular network for the one or more sample bone orientation to generate one or more X-ray dark field image signal normalization values (110).

5. A method according to claim 4, wherein providing a resolution image of the sample bone (108) at a resolution resolving the trabecular network comprises acquiring a resolution X-ray image using a micro-CT or a peripheral CT scanner.

6. A method according to any one of the claims 4 or 5, wherein providing said plurality of X-ray dark field images of the sample bone (104) comprises acquiring a plurality of X-ray dark field images of the sample bone using a grating interferometer based X-ray dark field imaging apparatus, said corresponding plurality of different sample bone orientations (103) being determined relative to a grating orientation of the X-ray dark field imaging apparatus.

7. A method according to claim 3, wherein providing the image of the sample bone (108) at a resolution such that the trabecular network can be resolved comprises providing a computer simulated sample bone comprising a trabecular network and wherein providing the plurality of X-ray dark field images of the sample bone (104) at the corresponding plurality of different sample bone orientations (103) comprises performing a plurality of numerical X-ray scattering simulations for the computer-simulated sample bone at a corresponding plurality of different computer-simulated sample bone orientations relative to a modelled X-ray dark field imaging apparatus, the plurality of X-ray dark field images of the computer-simulated sample bone being numerically recorded at an image resolution such that the trabecular network is not resolved.

8. A method according to any one of claims 3 to 7, wherein each of the plurality of X-ray dark field images of the sample bone corresponding to a single sample bone orientation is provided for a different position of the sample bone (102) with respect to an optical axis of an X-ray dark field imaging apparatus.

9. A computer program comprising instructions which, when the program is executed by a computer, cause the computer to carry out at least the signal conversion step of claim 1, and preferably also the rescaling step of claim 3.

10. A system (20) for expressing signals in a dark field X-ray image of bone in units of a trabecular quantity, comprising:
- an acquisition apparatus (30) for acquiring an X-ray dark field image of bone material (34; 44) having a trabecular network (41), the X-ray dark field image of the bone material being acquired at an image resolution such that the trabecular network is not resolved,
- a tracking unit (35) for tracking a position of the bone in the X-ray beam with respect to the acquisition apparatus, and
- at least one processing unit (36) operatively connected to the tracking unit and the acquisition apparatus to respectively receive as inputs therefrom a tracking signal for the bone material and the acquired X-ray dark field image of the bone material, the at least one processing unit being configured for
- extracting information regarding the position of the bone in the X-ray beam with respect to the acquisition apparatus from the received tracking signal,
- receiving a plurality of generated X-ray dark field image signal normalization values for a sample bone, and
- converting signals in the received X-ray dark field image of the bone material into a corresponding trabecular quantity, using the extracted position information of the bone material and the received plurality of generated X-ray dark field image signal normalization values.

11. A system according to claim 10, wherein the acquisition apparatus (30) comprises an X-ray imaging apparatus including an X-ray source (31), a grating interferometer (32a-c) and an X-ray detector (33), wherein the tracking unit (35) is tracking an orientation of the bone material (34; 44), when imaged by the X-ray imaging apparatus, relative to an orientation of the grating interferometer.

12. A system according to claim 10 or 11, wherein the tracking unit (35) is also tracking a position of the bone material (34; 44) with respect to an optical axis of the acquisition apparatus.

13. A system according to any one of the claims 10 to 12, wherein the tracking unit (35) comprises one or more of: a tracking camera for tracking in three dimensions, a tape measure, image processing means for extracting orientational and/or positional information from a reference structure in an acquired X-ray image, a bone support structure (39) generating a predetermined X-ray dark field signal when imaged by the acquisition apparatus.

14. A system according to the any one of the claims 12 or 13, wherein the at least one processing unit (36) is further adapted for rescaling signals in the acquired X-ray dark field image prior to converting the signals into a corresponding trabecular quantity, a degree of rescaling being determined by the position of the bone material with respect to an optical axis of the acquisition apparatus as tracked by the tracking unit.

15. A system according to any of the claims 10 to 14, further comprising a display unit (37) for displaying acquired X-ray dark field images in units of trabecular quantity and/or a storage unit (38) for storing a plurality of X-ray dark field image signal normalization values.
